(19) Europäisches Patentamt
European Patent Office
Office européen des brevets

(11) EP 0 567 848 B1

(12) **EUROPÄISCHE PATENTSCHRIFT**

(45) Veröffentlichungstag und Bekanntmachung des Hinweises auf die Patenterteilung:
**17.07.1996 Patentblatt 1996/29**

(51) Int. Cl.$^6$: **C07C 41/22**, C07D 317/46

(21) Anmeldenummer: **93106055.2**

(22) Anmeldetag: **14.04.1993**

# (54) Verfahren zur alpha-Chlorierung von Arylethern

Process for the alpha-chlorination of arylethern

Procédé pour la chloration d'aryléthers en position alpha

(84) Benannte Vertragsstaaten:
**BE CH DE FR GB IT LI NL**

(30) Priorität: **27.04.1992 DE 4213849**

(43) Veröffentlichungstag der Anmeldung:
**03.11.1993 Patentblatt 1993/44**

(73) Patentinhaber: **BAYER AG**
**51368 Leverkusen (DE)**

(72) Erfinder:
- **Marhold, Albrecht, Dr.**
  **W-5090 Leverkusen 1 (DE)**
- **Jelich, Klaus, Dr.**
  **W-5600 Wuppertal (DE)**

(56) Entgegenhaltungen:
**EP-A- 0 347 677** **CH-A- 675 122**
**CH-A- 676 119**

- **BULLETIN DE LA SOCIETE CHIMIQUE DE FRANCE Nr. 6, 1986, PARIS FR Seiten 925 - 929 BERNARD LANGLOIS ET AL. 'A Safe and Economical Synthesis of 3-(Trifluoro methoxy)aniline from 2-Chlorophenol'**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 567 848 B1

Printed by Rank Xerox (UK) Business Services
2.13.0/3.4

## Beschreibung

Die vorliegende Erfindung betrifft ein besonders vorteilhaftes Verfahren zur Chlorierung von Arylethern.

Es ist bereits bekannt, daß man Trichlormethoxybenzol herstellen kann, indem man Methoxybenzol in Tetrachlorkohlenstoff vorlegt, zum Sieden am Rückfluß erhitzt und dann Chlor einleitet (siehe Houben-Weyl, Methoden der organischen Chemie, Band E4, Seite 633 (1983)). Man kann Aryloxychlormethane auch aus Aryloxymethanen durch radikalisch initiierte Chlorierung herstellen, wobei man entweder in vorgelegtes Aryloxymethan Chlor einleitet oder ein Gemisch von Aryloxymethan und Sulphurylchlorid erhitzt (siehe DE-OS 3 821 130). Nachteilig bei diesen Verfahren ist, daß in verdünnten Lösungen gearbeitet wird, was ungünstige Raum-Zeit-Ausbeuten zur Folge hat und die Verwendung des wenig umweltverträglichen Tetrachlorkohlenstoffs als Lösungsmittel.

Es wurde nun ein Verfahren zur $\alpha$-Chlorierung von Arylethern der Formel (I) gefunden

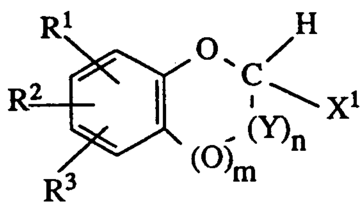

(I),

in der

$R^1$, $R^2$ und $R^3$ unabhängig voneinander jeweils für Wasserstoff, Fluor, Chlor, $CF_3$, $OCF_3$, $OCHF_2$, $OCF_2CF_2H$ oder $OCF_2Cl$ stehen,
$X^1$ für Wasserstoff, Fluor, Chlor oder $CF_3$ steht,
m für Null oder 1 steht und

im Falle m = Null, n für 1 und Y für Wasserstoff, Chlor oder Fluor und
im Falle m = 1, n für Null oder 1 und Y für $CH_2$, $CHCl$, $CCl_2$ oder $CF_2$ stehen,
das dadurch gekennzeichnet ist, daß man
Arylether der Formel (I) gleichzeitig mit Chlor in ein Reaktionsgefäß dosiert und bei Temperaturen im Bereich 60 bis 150°C arbeitet.

In das erfindungsgemäße Verfahren werden vorzugsweise Arylether der Formel (I) eingesetzt, bei denen

$R^1$ und $R^2$ unabhängig voneinander für Wasserstoff, Fluor, Chlor, $CF_3$ oder $OCF_3$ stehen,
$R^3$ für Wasserstoff steht,
$X^1$ für Wasserstoff, Fluor, Chlor oder $CF_3$ steht,
m für Null oder 1 steht und

im Falle m = Null, n für 1 und Y für Wasserstoff und
im Falle m = 1, n für Null oder 1 und Y für $CH_2$ oder $CF_2$ stehen.

Besonders bevorzugt werden Arylether der Formel (I) eingesetzt, bei denen

$R^1$ für Wasserstoff, Fluor oder Chlor steht,
$R^2$ und $R^3$ für Wasserstoff stehen,
$X^1$ für Wasserstoff oder $CF_3$ steht,
m für Null oder 1 steht und

im Falle m = Null, n für 1 und Y für Wasserstoff stehen und
im Falle m = 1, n für Null steht.

Es ist ein wesentliches Merkmal der vorliegenden Erfindung, daß man Arylether der Formel (I) gleichzeitig mit Chlor in ein Reaktionsgefäß dosiert. Man kann dabei beispielsweise so verfahren, daß man den gesamten einzusetzenden Arylether der Formel (I) oder den Hauptteil davon gleichzeitig mit Chlor in ein Reaktionsgefäß dosiert. Wenn man den Hauptteil des Arylethers gleichzeitig mit Chlor in ein Reaktionsgefäß dosieren will, kann man z.B. 0,5 bis 49 Gew.-% des insgesamt einzusetzenden Arylethers vorlegen und 99,5 bis 51 Gew.-% des insgesamt einzusetzenden Arylethers gleichzeitig mit dem Chlor zudosieren. Vorzugsweise legt man in solchen Fällen 5 bis 45 Gew.-% des insgesamt einzusetzenden Arylethers vor und dosiert 95 bis 55 Gew.-% des insgesamt einzusetzenden Arylethers mit dem

Chlor zu. Besonders bevorzugt legt man in diesen Fällen 8 bis 40 Gew.-% des insgesamt einzusetzenden Arylethers vor und dosiert 92 bis 60 Gew.-% des insgesamt einzusetzenden Arylethers gleichzeitig mit dem Chlor zu.

Vorzugsweise führt man das erfindungsgemäße Verfahren bei 65 bis 135°C, insbesondere bei 70 bis 120°C, durch.

Das erfindungsgemäße Verfahren kann man in Abwesenheit oder in Anwesenheit von Lösungsmittel durchführen.

Als Lösungsmittel für das erfindungsgemäße Verfahren kommen solche aus der Gruppe der Chlorierungsprodukte von Arylethern der Formel (I), von Fluorierungsprodukten der Chlorierungsprodukte von Arylethern der Formel (I) und der inerten aromatischen Lösungsmittel in Frage.

Chlorierungsprodukte der Arylether der Formel (I) entsprechen z.B. der Formel (II)

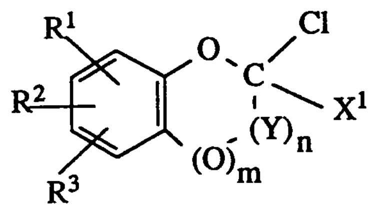

(II).

In der Formel (II) haben $R^1$, $R^2$, $R^3$, $X^1$, m, n und Y die gleiche Bedeutung wie bei Formel (I). $X^1$ steht hier vorzugsweise für Chlor und Y steht hier im Falle m = Null vorzugsweise für Chlor oder Fluor und im Falle m = 1 vorzugsweise für $CCl_2$ oder $CF_2$.

Als Lösungsmittel für das erfindungsgemäße Verfahren geeignete Chlorierungsprodukte von Arylethern der Formel (I) sind vorzugsweise Trichlormethoxybenzol und Trichlormethoxybenzole mit folgenden Substituenten: 2-Chlor, 3-Chlor, 4-Chlor, 2,3-Dichlor, 2,4-Dichlor, 2,5-Dichlor, 2,6-Dichlor, 2-Fluor, 3-Fluor, 4-Fluor, 2,4-Difluor, 2-Trifluormethyl, 3-Trifluormethyl, 4-Trifluormethyl, 2-Trifluormethyl-3-chlor, 2-Trifluormethyl-4-chlor, 2-Trifluormethyl-5-chlor, 4-Trifluormethyl-3-chlor und 2,3,4-Trifluormethoxy, sowie 2,2-Dichlorobenzodioxol und 2,2-Dichlorobenzodioxole mit folgenden Substituenten: 4-Chlor, 5-Chlor, 4-Fluor und 5-Fluor.

Chlorierungsprodukte von Arylethern der Formel (I) können auch Gemische verschiedener Verbindungen sein und/oder rohe und/oder isolierte Chlorierungsprodukte aus einem vorangegangenen Ansatz.

Häufig werden Chlorierungsprodukte von Arylethern der Formel (I) weiterverarbeitet, indem die Chloratome in $\alpha$-Position ganz oder teilweise mit Fluorierungsmitteln gegen Fluoratome ausgetauscht werden. So wird beispielsweise aus Trichlormethoxybenzol Trifluormethoxybenzol, Chlordifluormethoxybenzol und/oder Dichlorfluormethoxybenzol erhalten. Auch derartige Fluorierungsprodukte der Chlorierungsprodukte von Arylethern der Formel (I) können als Lösungsmittel für das erfindungsgemäße Verfahren eingesetzt werden. Solche Fluorierungsprodukte können z.B. als reine Produkte, im Gemisch untereinander, im Gemisch mit Chlorierungsprodukten von Arylethern der Formel (I) und/oder als rohe und/oder isolierte Produkte aus einer Fluorierung von Chlorierungsprodukten von Arylethern der Formel (I) eingesetzt werden.

Als inerte aromatische Lösungsmittel kommen beispielsweise Chlorbenzol, Dichlorbenzole, Benzotrifluorid, Chlorbenzotrifluoride, Bistrifluormethylbenzole, Bistrifluormethoxybenzole, Chlor-bistrifluormethylbenzole und Fluorchlorbenzole in Frage. Inerte aromatische Lösungsmittel können als solche, im Gemisch untereinander und/oder im Gemisch mit Chlorierungsprodukten von Arylethern der Formel (I) und/oder im Gemisch mit Fluorierungsprodukten von Chlorierungsprodukten von Arylethern der Formel (I) eingesetzt werden.

Wenn in das erfindungsgemäße Verfahren Lösungsmittel eingesetzt werden, so kann dies beispielsweise in Mengen von 5 bis 100 Gew.-%, bezogen auf insgesamt eingesetzten Arylether der Formel (I), geschehen Vorzugsweise beträgt diese Menge von 5 bis 50 Gew.-%, insbesondere von 5 bis 20 Gew.-%.

Die erfindungsgemäße Chlorierung wird mit elementarem Chlor durchgeführt. Man leitet dieses im allgemeinen unverdünnt gasförmig in das Reaktionsgemisch ein.

Man kann das erfindungsgemäße Verfahren im allgemeinen ohne einen Zusatz von Chlorierungskatalysatoren durchführen. Man kann gegebenenfalls auch Chlorierungskatalysatoren zusetzen. Beispiele hierfür sind Phosphortrichlorid, Phosphoroxychlorid, Phosphorpentachlorid, Kaliumchlorid und Mischungen davon. Man kann Chlorierungskatalysatoren z.B. in Mengen von 0 bis 15 Gew.-% (bezogen auf insgesamt eingesetzten Arylether der Formel (I)) verwenden.

Bei einer besonderen Ausführungsform der vorliegenden Erfindung arbeitet man in Gegenwart eines Radikalbildners oder von UV-Licht. Als Radikalbildner sind z.B. Benzoylperoxid, Diacetylperoxid, Succinylperoxid oder Azobisisobuttersäurenitril geeignet. Man kann Radikalbildner z.B. in Mengen von 0 bis 10 Gew.-% (bezogen auf insgesamt eingesetzten Arylether der Formel (I)) verwenden.

Man kann auch sowohl Chlorierungskatalysatoren kombiniert mit Radikalbildnern oder UV-Licht anwenden.

Das Ende der Chlorierungsreaktion und damit der Zeitpunkt für die Beendigung des Einleitens von Chlor kann man beispielsweise gaschromatographisch bestimmen.

Das nach Beendigung der Chlorierungsreaktion vorliegende Gemisch kann man beispielsweise zunächst mit einem inerten Gas, z.B. Stickstoff, ausblasen und gegebenenfalls anschließend durch Destillation das Lösungsmittel und gegebenenfalls das Reaktionsprodukt von höhersiedenden Nebenprodukten abtrennen.

Die erfindungsgemäß erhältlichen Produkte können beispielsweise mit Fluorierungsmitteln umgesetzt, und die dann erhältlichen Fluorverbindungen zur Herstellung von z.B. Pflanzenschutzmitteln verwendet werden. So ist beispielsweise Difluorbenzodioxol ein Zwischenprodukt für in der EP-OS 333 658 beschriebene Fungizide und für das Insektizid Alsystin® (CAS-Nr. 64 628-44-0).

Das erfindungsgemäße Verfahren hat eine Reihe von Vorteilen. So kann im Gegensatz zum Stand der Technik ohne Lösungsmittel oder in konzentrierteren Lösungen gearbeitet werden, was deutlich höhere Raum-Zeit-Ausbeuten zur Folge hat Weiterhin wird der wenig umweltverträgliche Tetrachlorkohlenstoff als Lösungsmittel vermieden. Schließlich ist die Verwendung der Chlorierungsprodukte des erfindungsgemäßen Verfahrens als Lösungsmittel oder die Verwendung von Fluorierungsprodukten der Chlorierungsprodukte technisch, verfahrenstechnisch und wirtschaftlich besonders vorteilhaft, da es sich um systemimmanente Lösungsmittel handelt.

## Beispiele

### Beispiel 1

In einer Chlorierungsapparatur mit Tauchlampe wurden 30 g 4-Chloranisol bei 80 bis 85°C vorgelegt, 5 g Phosphortrichlorid zugegeben und unten Bestrahlung mit UV-Licht Chlor eingeleitet. Mit dem Nachlassen der Chloraufnahme wurde weiteres 4-Chloranisol zudosiert und weiterhin Chlor eingeleitet Nachdem insgesamt 200 g 4-Chloranisol zugegeben wurden, wurden gaschromatographisch die Beendigung der Chlorierung überprüft und danach die Chlorzufuhr abgestellt. Nach dem Ausblasen mit Stickstoff wurde der Ansatz destilliert. Man erhielt 310 g 4-Chlor-trichlormethoxybenzol (Siedepunkt: 122-126°C/16 mbar, Brechungsindex $n_D^{20}$ : 1,5565), was einer Ausbeute von 89,8% der Theorie entspricht.

### Beispiel 2

Der auf einer Glasapparatur befindliche Tropftrichter wurde mit 70 g 4-Chloranisol, in dem 2 g Azobisisobuttersäurenitril gelöst waren, gefüllt. Nach Erhitzen des Reaktionskolbens, in dem 50 g 4-Chloranisol vorgelegt waren, auf 80°C wurde langsam der Inhalt des Tropftrichters zugetropft und gleichzeitig Chlor eingeleitet. Die Chlormenge wurde so reguliert, daß die Reaktionstemperatur 90°C nicht überstieg. Innerhalb von 9 Stunden wurde Chlor und Ausgangsmaterial so zudosiert, daß anfangs mehr Chlor zur Chlorierung der vorgelegten Menge eingeleitet wurde und gegen Ende die Chlormenge etwa der stöchiometrisch erforderlichen Menge (in Bezug auf die zudosierte Menge 4-Chloranisol) entsprach. Anschließend wurde mit Stickstoff ausgeblasen und der Ansatz destilliert. Es wurden 7 g eines Vorlaufs erhalten, der erneut in die Chlorierung eingesetzt werden konnte und 167 g 4-Chlortrichlormethoxybenzol. Der Rückstand betrug 21 g. Die isolierte Menge 4-Chlor-trichlormethoxybenzol entsprach 80,7% der Theorie.

### Beispiel 3

In einer Glasapparatur wurden 50 g 4-Chlor-trichlormethoxybenzol, 1 g Phosphortrichlorid und 1 g Kaliumchlorid vorgelegt. Der auf der Glasapparatur befindliche Tropftrichter wurde mit 120 g 4-Chloranisol und 2 g Azobisisobuttersäurenitril beschickt. Nach Erwärmung des Kolbeninhalts auf 80°C wurde der Inhalt des Tropftrichters langsam in das Reaktionsgefäß tropfen gelassen und gleichzeitig die Chlorzufuhr gestartet. Der weitere Arbeitsablauf war wie in Beispiel 2. Es wurden 219g 4-Chloranisol erhalten.

### Beispiel 4

Es wurde verfahren wie in Beispiel 3, mit dem Unterschied, daß anstelle des 4-Chlor-trichlormethoxybenzols 50 g 4-Chlor-trifluormethoxybenzol vorgelegt wurden. Nach der Destillation wurde 4-Chlor-trifluormethoxybenzol zusammen mit etwas 4-Chlor-dichlormethoxybenzol und 4-Chlor-trichlormethoxybenzol zurückgewonnen, die dem nächsten Ansatz zugeführt werden konnten. Die Ausbeute an 4-Chlor-trichlormethoxybenzol betrug 186g.

### Beispiel 5

In einer Chlorierungsapparatur aus Glas mit Chloreinleitung, Rückflußkühler, Gasableitung und Tropftrichter wurden in den Tropftrichter 200 g 2-Chloranisol und 5 g Azobisisobuttersäurenitril eingefügt. In den Kolben wurden 5 g Phosphortrichlorid vorgelegt, die auf 80°C erwärmt wurden. Anschließend wurde 50 g des Inhalts des Tropftrichters zulaufen gelassen und dann bei 80 bis 90°C Chlor zudosiert. Nach-dem etwa 60 g Chlor eindosiert waren, wurde

zusätzlich zu dem Chlor auch wieder weiteres Gemisch aus dem Tropftrichter zudosiert. Der Endpunkt der Chlorierung war nach 8 Stunden erreicht (gaschromatographische Kontrolle). Nach dem Ausblasen des Ansatzes mit trockenem Stickstoff wurde destilliert. Es wurden 310 g (= 89,8% der Theorie) 2-Chlor-trichlormethoxybenzol (Siedepunkt 126-130°C/16 mbar) und 40g acetonlöslicher Rückstand erhalten.

Beispiel 6

Es wurde verfahren wie in Beispiel 5, jedoch wurden im Kolben 50 g 2-Chlorbenzotrifluorid zusammen mit 5 g Phosphortrichlorid vorgelegt. Die Dosierung und Chlorierung erfolgte im übrigen analog. Durch Destillation wurde das Lösungsmittel zurückgewonnen und ein Destillat von 309 g 2-Chlor-trichlormethoxybenzol erhalten. Das zurückgewonnene Lösungsmittel enthielt noch Anteile von unvollständig chloriertem Material und konnte erneut eingesetzt werden.

Beispiel 7

In 100 ml 1,3-Bistrifluormethylbenzol wurden 0,5 g Kaliumchlorid und 10 g Difluormethoxybenzol vorgelegt und anschließend bei 100°C unter UV-Bestrahlung Chlor eingeleitet. Innerhalb 1 Stunde wurden weitere 20 g Difluormethoxybenzol zudosiert. Nach Ende der Chloraufnahme wurde mit Stickstoff ausgeblasen und anschließend destilliert. Es wurden 35,5 g Chlordifluormethoxybenzol erhalten (Siedepunkt 144°C, Brechungsindex $n_D^{20}$: 1,4479).

Beispiel 8

Es wurde wie in Beispiel 7 verfahren, jedoch wurde anstelle des Difluormethoxybenzols 100 g 2,2,2-Trifluorethoxybenzol eingesetzt. Davon wurden 10 g vorgelegt und die restlichen 90 g gemeinsam mit dem Chlor zudosiert. Es wurden 101,5 g 1,1-Dichlor-2,2,2-trifluorethoxybenzol erhalten (Siedepunkt 77-80°C/20 mbar, Brechungsindex $n_D^{20}$: 1,4615).

Beispiel 9

In einer Rührapparatur mit Chloreinleitung, Dosiervorrichtung, Intensivkühler und Gasableitung wurden 600 g Chlorbenzol bei 110°C vorgelegt und anschließend eine Lösung von 18 g Azobisisobuttersäurenitril in 900 g Benzodioxol langsam zudosiert (75 bis 80 g pro Stunde) und gleichzeitig 100 bis 105 g Chlor je Stunde eingeleitet. Die Reaktion war exotherm und wurde durch Entfernung des Heizbades bei 110°C gehalten. Nach vollständiger Eindosierung von Benzodioxol und Chlor wurde noch für weitere 30 Minuten Chlor eingeleitet, anschließend mit Stickstoff ausgeblasen und destilliert. Nach der Abnahme von 630g einer ersten Fraktion (Chlorbenzol und erneut einsetzbares Chlorierprodukt) wurden 1240 g 2,2-Dichlorbenzodioxol erhalten (Siedepunkt 81-90°C/12 mbar).

Beispiel 10

In einer Rührapparatur mit Chloreinleitung, Dosiervorrichtung, Intensivkühler und Gasableitung wurden 60 g Dichlorbenzodioxol bei 130°C vorgelegt und anschließend eine Lösung von 1,8 g Azobisisobuttersäurenitril in 122 g Benzodioxol innerhalb von 3 Stunden zudosiert und gleichzeitig 150 g Chlor eingeleitet Nach dem Ende der Dosierung wurde bei 130°C mit Stickstoff ausgeblasen. Es wurden 228 g 2,2-Dichlorbenzodioxol vom Siedepunkt 81-90°C bei 12 mbar erhalten.

Das Rohprodukt der Chlorierung (250 g) konnte ohne weitere Reinigung in eine Fluorierung zur Herstellung von 2,2-Difluorbenzodioxol eingesetzt werden.

Beispiel 11

490 g 3,5-Bis-fluormethyl-phenol wurden in 1 000 ml Aceton zusammen mit 50 g 50 gew.-%iger wäßriger Natronlauge vorgelegt und bei 25°C bis zur Sättigung Hexafluorpropen eingeleitet. Anschließend wurde der Ansatz mit 1 l Wasser verdünnt, die organische Phase abgetrennt, getrocknet und destilliert. Es wurden 673 g 3,5-Bis-fluormethyl-phenyl-1,1,2,3,3,3-hexafluorpropylether mit einem Siedepunkt von 65 bis 70°C bei 22 mbar und einem Brechungsindex $n_D^{20}$ von 1,3560 erhalten.

Das so erhaltene Produkt wurde 3 Stunden lang bei Rückflußtemperatur unter UV-Bestrahlung mit Chlor behandelt. Bei der anschließenden gaschromatografischen Untersuchung wurden keine Chlorierungsprodukte gefunden.

3,5-Bis-fluormethyl-phenyl-1,1,2,3,3,3-hexafluorpropylether ist deshalb als Lösungsmittel für das erfindungsgemäße Verfahren zur $\alpha$-Chlorierung von Arylethern geeignet.

Beispiel 12

In 500 ml 1,4-Bis-fluormethylbenzol wurden 200 g 2,3-Dichlor-benzo-1,4-dioxen vorgelegt und auf 110°C erhitzt. Unter UV-Bestrahlung wurde Chlor eingeleitet und der Fortgang der Chlorierung mittels gaschromatografischer Analyse kontrolliert. Nach vollständigem Umsatz wurde die Chlorzufuhr unterbrochen und das Reaktionsgefäß mit Stickstoff ausgeblasen. Die Destillation ergab 240 g 2,2,3,3-Tetrachlor-benzo-1,4-dioxen mit einem Siedepunkt von 140 bis 142°C bei 20 mbar und einem Schmelzpunkt von 88 bis 92°C.

**Patentansprüche**

1. Verfahren zur α-Chlorierung von Arylethern der Formel (I)

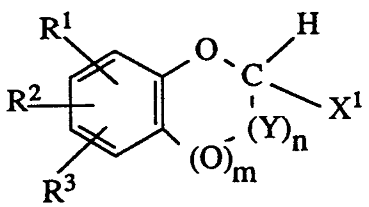

(I),

in der

$R^1$, $R^2$ und $R^3$ unabhängig voneinander jeweils für Wasserstoff, Fluor, Chlor, $CF_3$, $OCF_3$, $OCHF_2$, $OCF_2CF_2H$ oder $OCF_2Cl$ stehen,
$X^1$ für Wasserstoff, Fluor, Chlor oder $CF_3$ steht,
m für Null oder 1 steht und

im Falle m = Null, n für 1 und Y für Wasserstoff, Chlor oder Fluor und
im Falle m = 1, n für Null oder 1 und Y für $CH_2$, CHCl, $CCl_2$ oder $CF_2$ stehen,
dadurch gekennzeichnet, daß man Arylether der Formel (I), gleichzeitig mit Chlor in ein Reaktionsgefäß dosiert und bei Temperaturen im Bereich 60 bis 150°C arbeitet.

2. Verfahren nach Anspruch 1, dadurch gekennzeichnet, daß man Arylether der Formel (I) einsetzt, bei denen

$R^1$ und $R^2$ unabhängig voneinander für Wasserstoff, Fluor, Chlor, $CF_3$ oder $OCF_3$ stehen,
$R^3$ für Wasserstoff steht,
$X^1$ für Wasserstoff, Fluor, Chlor oder $CF_3$ steht,
m für Null oder 1 steht und

im Falle m = Null, n für 1 und Y für Wasserstoff und
im Falle m=1, n für Null oder 1 und Y für $CH_2$ oder $CF_2$ stehen.

3. Verfahren nach Ansprüchen 1 und 2, dadurch gekennzeichnet, daß man den gesamten einzusetzenden Arylether der Formel (I) gleichzeitig mit Chlor in ein Reaktionsgefäß dosiert.

4. Verfahren nach Ansprüchen 1 bis 2, dadurch gekennzeichnet, daß man 0,5 bis 49 Gew.-% des insgesamt einzusetzenden Arylethers vorlegt und 99,5 bis 51 Gew.-% des insgesamt einzusetzenden Arylethers gleichzeitig mit dem Chlor zudosiert

5. Verfahren nach Ansprüchen 1 bis 4, dadurch gekennzeichnet, daß man es in Abwesenheit von Lösungsmitteln oder in Anwesenheit von Chlorierungsprodukten von Arylethern der Formel (I), von Fluorierungsprodukten der Chlorierungsprodukte von Arylethern der Formel (I) oder inerten aromatischen Lösungsmitteln durchführt.

6. Verfahren nach Ansprüchen 1 bis 5, dadurch gekennzeichnet, daß man es ohne einen Zusatz von Chlorierungskatalysatoren durchführt.

7. Verfahren nach Ansprüchen 1 bis 6, dadurch gekennzeichnet, daß man es in Gegenwart von 5 bis 100 Gew.-% Lösungsmittel, bezogen auf insgesamt eingesetzten Arylether der Formel (I), durchführt.

8. Verfahren nach Ansprüchen 1 bis 6, dadurch gekennzeichnet, daß man es in Gegenwart von Phosphortrichlorid, Phosphoroxychlorid, Phosphorpentachlorid, Kaliumchlorid oder Mischungen davon als Chlorierungskatalysatoren durchführt.

9. Verfahren nach Ansprüchen 1 bis 8, dadurch gekennzeichnet, daß man es in Gegenwart eines Radikalbildners oder von UV-Licht durchführt.

10. Verfahren nach Ansprüchen 1 bis 9, dadurch gekennzeichnet, daß man den Zeitpunkt für die Beendigung des Einleitens von Chlor, gaschromatographisch bestimmt.

## Claims

1. Process for the α-chlorination of aryl ethers of formula (I):

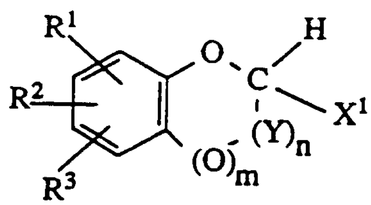

(I)

in which

$R^1$, $R^2$ and $R^3$ independently of one another are in each case hydrogen, fluorine, chlorine, $CF_3$, $OCF_3$, $OCHF_2$, $OCF_2CF_2H$ or $OCF_2Cl$,

$X^1$ is hydrogen, fluorine, chlorine or $CF_3$,

m is zero or 1 and

in the case where m = zero, n is 1 and Y is hydrogen, chlorine or fluorine, and
in the case where m = 1, n is zero or 1 and Y is $CH_2$, CHCl, $CCl_2$ or $CF_2$,
characterised in that aryl ethers of formula (I) are metered into a reaction vessel at the same time as chlorine, the reaction being carried out at temperatures in the range from 60 to 150°C.

2. Process according to Claim 1, characterised in that the aryl ethers used are those of formula (I) in which

$R^1$ and $R^2$ independently of one another are hydrogen, fluorine, chlorine, $CF_3$ or $OCF_3$,

$R^3$ is hydrogen,

$X^1$ is hydrogen, fluorine, chlorine or $CF_3$,

m is zero or 1 and

in the case where m = zero, n is 1 and Y is hydrogen, and
in the case where m = 1, n is zero or 1 and Y is $CH_2$ or $CF_2$.

3. Process according to Claims 1 and 2, characterised in that all the aryl ether of formula (I) to be used is metered into a reaction vessel at the same time as chlorine.

4. Process according to Claims 1 and 2, characterised in that 0.5 to 49% by weight of the total amount of aryl ether to be used is introduced first and 99.5 to 51% by weight of the total amount of aryl ether to be used is metered in at the same time as the chlorine.

5. Process according to Claims 1 to 4, characterised in that it is carried out in the absence of solvents or in the presence of chlorination products of aryl ethers of formula (I), fluorination products of chlorination products of aryl ethers of formula (I), or inert aromatic solvents.

6. Process according to Claims 1 to 5, characterised in that it is carried out without the addition of chlorination catalysts.

7. Process according to Claims 1 to 6, characterised in that it is carried out in the presence of 5 to 100% by weight of solvent, based on the total amount of aryl ether of formula (I) used.

8. Process according to Claims 1 to 6, characterised in that it is carried out in the presence of phosphorus trichloride, phosphorus oxychloride, phosphorus pentachloride, potassium chloride or mixtures thereof as chlorination catalysts.

9. Process according to Claims 1 to 8, characterised in that it is carried out in the presence of a radical-forming agent or UV light.

10. Process according to Claims 1 to 9, characterised in that the point at which the introduction of chlorine is ended is determined by gas chromatography.

**Revendications**

1. Procédé pour la chloruration en alpha des éthers aryliques de formule I

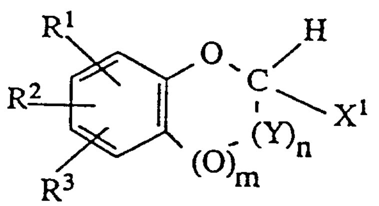

(I),

dans laquelle
$R^1$, $R^2$ et $R^3$ représentent chacun, indépendamment les uns des autres, l'hydrogène, le fluor, le chlore, $CF_3$, $OCF_3$, $OCHF_2$, $OCF_2CF_2H$ ou $OCF_2Cl$.
$X^1$ représente l'hydrogène, le fluor, le chlore ou $CF_3$,
m est égal à 0 ou 1 et
lorsque m = 0, n = 1 et Y représente l'hydrogène, le chlore ou le fluor, et
lorsque m = 1, n = 0 ou 1 et Y représente $CH_2$, CHCl, $CCl_2$ ou $CF_2$,
caractérisé en ce que l'on introduit simultanément l'éther arylique de formule I et le chlore dans un récipient de réaction et on opère à des températures dans l'intervalle de 60 à 150°C.

2. Procédé selon revendication 1, caractérisé en ce que l'on met en oeuvre des éthers aryliques de formule I dans laquelle
$R^1$ et $R^2$ représentent chacun, indépendamment l'un de l'autre, l'hydrogène, le fluor, le chlore, $CF_3$ ou $OCF_3$,
$R^3$ représente l'hydrogène,
$X^1$ représente l'hydrogène, le fluor, le chlore ou $CF_3$,
m est égal à 0 ou 1 et
lorsque m = 0, n = 1 et Y représente l'hydrogène et
lorsque m = 1, n = 0 ou 1 et Y représente $CH_2$ ou $CF_2$.

3. Procédé selon les revendications 1 et 2, caractérisé en ce que l'on introduit simultanément la totalité de l'éther arylique de formule I mis en oeuvre et le chlore dans un récipient de réaction.

4. Procédé selon les revendications 1 à 2, caractérisé en ce que l'on introduit au départ dans l'appareil, de 0,5 à 49 % du poids total d'éther arylique à mettre en oeuvre et on ajoute de 99,5 à 51 % du poids total de l'éther arylique mis en oeuvre simultanément avec le chlore.

5. Procédé selon les revendications 1 à 4, caractérisé en ce que l'on opère en l'absence de solvant ou en présence de produits de chloruration d'éthers aryliques de formule I, de produits de fluoruration de produits de chloruration d'éthers aryliques de formule I ou de solvants aromatiques inertes.

6. Procédé selon les revendications 1 à 5, caractérisé en ce que l'on opère en l'absence de catalyseur de chloruration.

7. Procédé selon les revendications 1 à 6, caractérisé en ce que l'on opère en présence de 5 à 100 % en poids de solvant par rapport au poids total de l'éther arylique de formule I mis en oeuvre.

8. Procédé selon les revendications 1 à 6, caractérisé en ce que l'on opère en présence de trichlorure de phosphore, d'oxychlorure de phosphore, de pentachlorure de phosphore, de chlorure de potassium ou de leurs mélanges, servant de catalyseurs de chloruration.

9. Procédé selon les revendications 1 à 8, caractérisé en ce que l'on opère en présence d'un inducteur radicalaire ou de lumière UV.

10. Procédé selon les revendications 1 à 9, caractérisé en ce que l'on détermine par chromatographie en phase gazeuse le moment où l'on doit interrompre l'injection de chlore.